# EUROPEAN PATENT APPLICATION

(11) **EP 3 878 415 A1**
(43) Date of publication of application: **15.09.2021**
(21) Application number: 20315034.7
(22) Date of filing: 09.03.2020
(51) Int. Cl.: A61F 5/00

(54) **A DEVICE FOR TREATMENT OF OBESITY OR DIABETES OF A PATIENT AND A METHOD FOR SELECTING SUCH A DEVICE**

(71) Applicant: BariaTek Medical, 75003 Paris (FR)
(72) Inventor: MANOS, Thierry, 13007 Marseille (FR); BASTID, Christophe, 13009 Marseille (FR); POULETTY, Philippe, 75005 Paris (FR)
(74) Representative: Hepp Wenger Ryffel AG

(57) **Abstract**

A device (10) for treatment of obesity or diabetes comprises a duodenal tube (12), first anchor (14) arranged at a pre-defined distance (D) from a proximal end (15) of the duodenal tube (12). The first anchor (14) is adapted for anchoring the tube (12) distally to the pylorus (P) without mucosal involvement. A second anchor (17) in the form of a conical, inflatable balloon is used for anchoring the device (10) in the stomach (S) of the patient. The duodenal tube (12) is flexible and can be arranged in the duodenum (D) and in the jejunum (J) of the patient.

## Description

The invention is directed to a device for the treatment of obesity or diabetes of a patient and to a method for selecting such a device.

It is known in the prior art to use implantable devices for the treatment of obesity which bypass a certain length of the duodenum. Typically such devices may be delivered in a minimally invasive manner and are anchored at one or more positions.

US 2018/0214293 discloses the anchoring of a device on the pylorus with stents or alternatively by using an inflatable balloon. A similar anchoring is disclosed in US 9,421,116.

WO 2014/195954 discloses a device with several anchors. First and second anchors comprise stents and are pushed against the walls of parts of the duodenum. An additional, intragastric anchor for deployment within the stomach of the patient is disclosed. WO 2012/087669 also discloses intragastric anchors.

US 2005/273060 discloses the anchoring of a device on the pylorus with balloons which also reduce the volume of the stomach.

US 5,820,584 also discloses the anchoring of a device on both sides of the pylorus. The anchoring of a device for treatment of obesity and diabetes on the pylorus by means of inflatable anchors is also disclosed in US 2011/0004320.

US 2017/312112 discloses a transpyloric device for accepting chyme from the stomach and conducting said chyme in a bypass like manner through a patients duodenum. The device is held in place by balloon segments which sit on a transpyloric conducting element.

The devices according to the prior art do all have certain disadvantages. In particular, they may be difficult to deploy and/or anchor, they may create an undesirable mucosal engagement and they may have unreliable anchoring.

It is an object of the present invention to overcome the drawbacks of the prior art, in particular to provide a device for treatment of obesity or diabetes which is easy to manufacture, easy to deploy, which offers a reliable anchoring and which does not have negative side effects, in particular due to mucosal engagement. A further object of the invention is to provide a method which allows to improve the treatment of the patient.

According to the present invention, these and other objects are solved with a device and a method according to the independent claims.

According to the invention, a device for treatment of obesity or diabetes is provided. Typically, with some minor modifications, the same principle of a device can be used for both, the treatment of obesity and the treatment of diabetes. The device comprises a duodenal tube. This tube is adapted to be placed in the duodenum and optionally in the jejunum or also in the ileum of a patient. A first anchor is arranged at a pre-defined distance from a proximal end of the duodenal tube. This first anchor is preferably adapted for anchoring the tube distally to the pylorus without substantial mucosal involvement, in particular without mucosal penetration. In this context, without substantial mucosal involvement means that even though the mucosa may occasionally or temporarily be contacted by the first anchor, anchoring of the device is not based on a contact, engagement or penetration of the mucosa. More particularly, the anchor is formed in such a way that it does not continuously and tightly contact the mucosa. By providing such an anchor, irritations of the mucosa may be avoided. Typically, the first anchor is designed such as to provide a certain weight which will create a positioning and anchoring due to peristatic effects. Since anchoring is not due to frictional engagement, the size and shape of the first anchor may be chosen such as to avoid mucosal engagement.

In a preferred embodiment the first anchor may comprise or may be formed of an expandable structure. Typically, self-expandable structures are known in the art for providing stents. The self-expandable structure can be made of a metal, preferably of a shape memory metal such as Nitinol. The self-expandable structure preferably can be braided. Other embodiments such as embodiments using self-expandable structures which are cut from a tube are also conceivable. It is also possible to use balloon expandable structures or structures which are self-expandable to a certain extent, but need balloon support for a complete expansion. Other biocompatible materials such as other metals, alloys or biocompatible plastic materials are possible.

The expandable structure may be covered by one or more layer of a covering material, in particular a polymeric layer including or made of Dacron or a biologic layer, such as pericardial tissue. The layer may be a foil of a plastic material. It may also be a woven or knitted structure of polymeric filaments.

The first anchor and in particular the first expandable structure may be retrievable and repositionable. A retrievable structure may be brought back into a narrower and typically into the initial configuration, allowing removal of the structure and of the entire device from the patient's body. This allows easy removal of the device, e.g. in case of unexpected side effects. The structure and hence the device also can be repositionable, i.e. allowing reduction of the size allowing to displace the device at the application site, followed by another expansion for anchoring at another site.

Any mechanism for bringing the structure and the device into a narrower configuration may be possible: Interior engagement members may allow engagement with an internal tool for crimping from the inside. Deactivation of a support structure such as an inflated balloon may lead to a collapse of an expanded structure in case the structure was expanded against a radial force, e.g. an elastic deformation. It is also conceivable to use thermal or chemical mechanisms for bringing the structure back to the narrower configuration.

It is also conceivable to additionally or alternatively anchor the duodenal tube by means of patches which are attachable to the mucosa and which are linked to the duodenal tube through connections, e.g. threads, fibres or wires. The patch preferably comprises a biocompatible adhesive. It alternatively or additionally may be adapted to promote cell biocolonization. The patches also can be biodegradeable. Such patches may help to further anchor the device, without having, however, any traumatic effect on the mucosa.

The layers and/or the patches may be formed such as to avoid endothelialisation.

According to a particularly preferred embodiment of the invention, the duodenal tube may be provided with a second anchor which is arranged at its proximal end. The second anchor is adapted to be positioned proximally of the pylorus for anchoring the device in the stomach. The size and the shape of this second anchor is chosen such as to avoid mucosal engagement.

The second anchor may be formed as an inflatable balloon which is adapted to be positioned within the patient's stomach such as to reduce the gastric functional volume. The balloon may be shaped and sized such as to avoid a tight contact with the inner wall of the stomach when it is appropriately inflated. In a first variant, the balloon may have an annular shape surrounding the duodenal tube. Thereby, a homogenous anchoring may be achieved. Furthermore, a regular, annular shape which completely surrounds the duodenal tube may lead to regular closure pattern of the passage into the duodenum.

In a first variant, the balloon may have a crown shape when it is inflated.

In a particularly preferred variant, the balloon may have a toroidal shape.

According to another variant, the balloon may have, in the inflated condition, a specific shape and size in an area neighbouring the connection to the duodenal tube: the inflated balloon may e.g. have a conical outer shape or a concave outer shape in a cross-section through a plane running through an axis of the device. The balloon may also have a tulip shape. These shapes combine a reliable anchoring and avoid a distal migration of the device without, however, continuously requiring an engagement of the anchor with tissue and in particular mucosal engagement.

By using a conical, concave or tulip shape, it is possible to place the device closer to the pylorus without the risk of contacting tissue.

Typically, the balloon may be adapted to be inflated to a volume of between 200 ml to 800 ml, more preferably to 300 ml to 450 ml.

While it is understood that such kind of second anchors are particularly preferred in combination with a device with a first anchor as described herein above, the skilled person will appreciate that such second anchor also can be used without such first anchor or with a differently shaped first anchor.

The second anchor additionally or alternatively may comprise a second expandable, in particular self-expandable structure. Such a device in particular may decrease the absorption of sugars and lipids and may be particularly suitable for treatment of diabetes.

The second anchor may have an at least partial hour-glass shape so that, preferably together with the first anchor, there may be an hour-glass like shape for anchoring on both sides of the pylorus. The hour glass shape may be symmetric or asymmetric.

Also this expandable structure may be made of a metal, preferably a shape memory metal such as Nitinol. Also the second expandable structure may be braided or alternatively laser-cut from a metallic tube. The second expandable structure also may be covered by one or more layers, in particular a polymeric layer including or made of Dacron or polyurethane or a biologic layer, such as pericardial tissue. The layer may be a foil of a plastic material. It may also be a woven or knitted structure of polymeric filaments.

In a similar way as described in context with the first anchor, also the second anchor may be retrievable and/or repositionable.

The second anchor may also comprise patches, similar as described above in context with the first anchor.

According to a preferred embodiment of the invention, the device may be provided with at least one element on the duodenal tube in addition to the anchors. Such element may have several purposes. It can be used for imaging if it is of metal or other radiopaque material and provides a higher contrast in e.g. x-ray imaging or in a CT scan. If made from metal or other materials with a high specific weight, it can also be used for additionally anchoring the device due to its weight.

In one embodiment the at least one element is metallic and can be formed as a ring which is mounted on the surface of the duodenal tube. Typically, the ring can be mounted at the distal end of the duodenal tube. Additionally or alternatively it can also be mounted in an area of the duodenal tube which is distant from the distal end. Typically, it can be arranged at a distance of 8 to 12cm from the distal end of the duodenal tube.

According to still another embodiment of the invention, the duodenal tube may be adapted to be shortened for adaptation to at least one characteristic of the patient. For this purpose, the tube in particular can be provided with markings which indicate a certain length and/or with weakening zones which facilitate shortening. Depending on certain characteristics of the patient to be treated, shorter or longer tubes may be selected for implantation. The characteristics typically may be the thickness of abdominal fat panicle, the fat mass surrounding the abdominal cavity, the visceral fat mass or the fat inside and/or outside the abdominal cavity. Computerized Tomography may be used in a non-invasive, objective and easy to repeat evaluation. Other characteristics may be the body surface area, the body mass index or the abdominal perimeter. By providing such an adaptation, an optimal treatment may be achieved. In particular, the amount of the effect created by the bypass may be individually chosen.

According to another preferred embodiment of the invention, the device can be activatable in dependence of a contact with the content of the stomach and or intestine. For this purpose, at least one of the duodenal tube, the first and the second anchor may be activated, e.g. expanded, if brought in contact with body fluid or nutrients. In particular, the duodenal tube may be dilated in response to a contact with contents of the stomach or intestine. In particular, the device hence may only become dilated if needed, i.e. if the stomach or intestine is filled.

Typically, the first anchor has an axial length of 1 to 3 cm and the second anchor has an axial length of 2 to 10 cm.

Preferably, the duodenal tube is made of a material which avoids a contact of nutrients migrating within the tube with the duodenal wall. Typically, the duodenal tube may be made of polyurethane. Any other material having a suitable anti-migrating effect on nutrients may be used. Furthermore it is possible to select a material for the duodenal tube which is reactive to the content of the stomach or intestine: In particular the material of the duodenal tube may have an osmotic permeability for specific nutrients which may decrease the higher the content of the nutrients is. It is also possible to choose a material which selectively reduces the retention or absorption of certain constituents within the duodenum. The material may e.g. be selectively permeable for fats, proteins or sugars, depending on the desired treatment.

According to another preferred embodiment, the duodenal tube may include sensors and/or actors for actively modifying the structure and/or the shape and/or the size of the duodenal tube in reaction to changing conditions. In particular, there may be actor on the device which modifies the shape of the tube in answer to the amount or type of bodily fluids measured by the sensors. For this purpose, the device may include electronics for treatment of signals provided by the sensors and for controlling an actuator, e.g. voltages influencing the structure of the material of the duodenal tube or driving members for changing the shape or the size such as e.g. piezo electric elements.

It is also possible to use sensors, in particular biosensors, which provide information, e.g. relating to such as the content of nutrients or the status, shape or size of the device. Such information may be used within the device in a closed loop feedback and/or may be transmitted externally, e.g. via wireless communications for subsequent use by a care person. In particular, it may be possible to selectively change the size and shape of the device in answer to the amount of nutrients measured in the stomach and/or in the intestine. In particular, in case of a higher amount of nutrients, the volume occupied by the device and/or the surface of the stomach and/or the intestine covered by the device may be increased.

While it is understood that specific adaptive or selective materials or mechanisms for the device and in particular the duodenal tube are particularly advantageous in context with a device with anchors as described herein above, it is understood that such materials or mechanisms can be used in context with any other implantable device for treatment of obesity or diabetes having a duodenal tube adapted to be placed in the duodenum and optionally in the jejunum or ileum of a patient.

The duodenal tube may have a length in the range of 300 to 800 mm, preferably 400 to 700 mm. It may have a diameter in the range of 20 to 35 mm. In a particularly preferred embodiment, the tube has a length of about 600 mm and a diameter of about 28 mm.

Another aspect of the invention is directed to a method for selecting a device for treatment of obesity or diabetes of a patient. In particular, the method is used for selecting a device as it has been described herein above.

In a first step at least one characteristic of the patient is determined. This characteristic may be the thickness of the abdominal fat panicle, the fat mass surrounding the abdominal cavity, the visceral fat mass or the fat inside and/or outside the abdominal cavity. The characteristic may also be the body surface are, the body mass index or the abdominal perimeter, measured on a standing patient at the umbilicus. The characteristic further may be the elasticity of the stomach, an index of elasticity of the stomach or evaluation tests of absorption of nutrients. By way of this, the patient specific absorption e.g. of fats may be taken into consideration. Of course, several of these characteristics can also be used in combination with each other.

On the basis of the selected characteristic or characteristics, an appropriate length of the duodenal tube is then defined.

In a final step, a device with a duodenal tube having the defined length is provided. This can be done by either shortening of the duodenal tube to the defined length or by selecting a device having a duodenal tube with the defined length. Such selection may be made either by selecting the device out of a set of devices having a various lengths or by individually manufacturing a device having the selected length.

Alternatively or additionally to the step of defining the length of a duodenal tube, the filling volume of a gastric anchoring balloon may be defined based on the determined characteristic or characteristics.

According to a preferred embodiment of the invention, the fat inside and/or outside the abdominal cavity may be determined by computerised Tomodensitometry.

This method provides for an individualised and optimised device for treatment specifically for the individual patient.

The invention will now be described with reference to certain embodiments and the accompanying drawings which show:
- Figure 1:: A schematic representation of a first embodiment of the invention
- Figure 2:: A schematic representation of a second embodiment of the invention
- Figure 3:: A representation of a third embodiment of the invention
- Figure 4:: The device according to figure 3 deployed within a patient
- Figure 5:: A schematic representation of a fourth embodiment of the invention
- Figure 6:: The device of figure 5 deployed in a patient
- Figure 7:: A flow-chart showing the steps of a method according to the invention
- Figure 8:: A schematic representation of a fifth embodiment of the invention and
- Figure 9: CT images for evaluation of the abdominal fat.

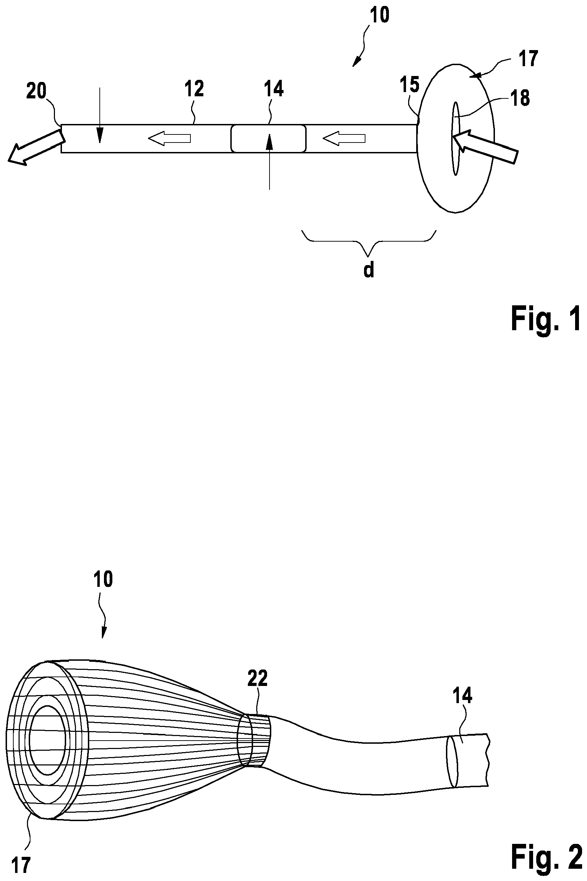

Figure 1 discloses a first embodiment of a device 10 which is used for treating a patient suffering from obesity. The device 10 is primarily formed by a duodenal tube 12 which has a second anchor 17 attached to its proximal end 15. Arranged at a distance d from the proximal end 15 there is a first anchor 14. The first anchor 14 is formed as a mass which has a tendency to move the duodenal tube 12 distally within the duodenum. This movement can be caused by peristaltic and/or gravitational effects. Second anchor 17 avoids a too far distal migration of the device 10.

The second anchor 17 is formed in the shape of a torus and surrounds the perimeter of the duodenal tube 12 neighbouring its proximal end 15. An entry opening 18 is arranged in the toroidal second anchor 18. Nutrients may enter the interior of the duodenal tube 12 from the stomach through the opening 18, as indicated with an arrow in figure 1. The nutrients then quit the duodenal tube 12 at its distal end 20, again indicated by an arrow. Thereby, a certain distance of the duodenum is bypassed and nutrients are prevented from contacting the wall of the duodenum.

In the present embodiment, the duodenal tube has a length of 600 mm and a diameter of 28 mm and is integrally formed with the balloon-like second anchor 17. It is formed of polyurethane. The mass 14 preferably is formed of a self-expandable, braided structure of wires of a shape memory material. The second anchor 17 comprises an entry opening (not shown) which is connectable with a device for inflation which allows to inflate the balloon to an appropriate volume and size, typically to a volume of 350 ml.

Figure 2 shows an alternative embodiment of the second anchor 17. In difference to the embodiment of figure 2, the second anchor 17 is not formed as a torous, but rather has a tulip shape, i.e. a shape which, in a cross-section through a plane running through an axis of the device is slightly convex.

In the embodiment shown in figure 2, the first anchor 14 is designed as a self-expandable structure made of braided nitinol wires. An additional support structure 22 is disclosed which helps to stabilize the device and/or anchor the device.

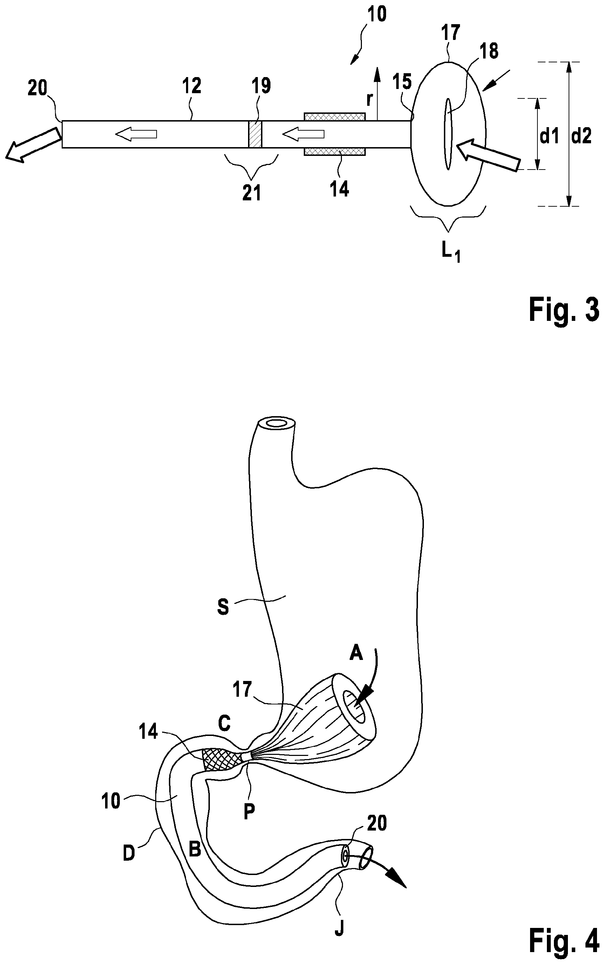

Figure 3 shows a more specific embodiment of a device 10 for treating a patient with obesity. The device is partly similar to the device of figure 1, with the following differences: the first anchor 14 is formed as a self-expandable structure made of braided nitinol. The embodiment of figure 3 is formed of the duodenal tube 12 made of polyurethane and of the inflatable second anchor 17. The second anchor 17 is attached to the duodenal tube 2 by gluing and is made of silicon. The second anchor 17 may have a toroidal, conical or tulip shape. The second anchor 17 has an axial length 11 of about 50mm, an internal diameter d1 of the opening 18 of about 28mm and an external diameter d2 of about 50mm. The lips of the second anchor 17 typically may have a radial length r of 10 to 25 mm. By appropriately selecting the size, thickness and/or material of the duodenal tube 12, it can be made sure that the tube is sufficiently flexible such as to adopt its shape to the shape of the duodenum D and optionally the jejunum J.

The device 10 deployed within the patient is shown in figure 4. The device is anchored proximately of the pylorus P by means of the second anchor 17 whereas it is anchored distally of the pylorus P by means of the first anchor 14. The second anchor 17 has a conical or tulip-like shape which avoids contact with the mucosal wall of the stomach S. The second anchor 17 has a double function. On the one hand it reduces the volume of the stomach for reducing the feeling of hunger of the patient. Additionally, it avoids migration of the device into the distal direction. Due to the conical or tulip shape, a permanent contact with the mucosal wall is, however, avoided. The second anchor 17 may freely float within the stomach. The first anchor 14 is also sized and shaped such that mucosal contact can be avoided. It is made sufficiently short, typically with a length of 10 mm to 25 mm and covered by a layer of made of a biocompatible material such as Dacron and having a thickness of 0,7mm to 2 mm, which is not shown in detail in figures 3 and 4.

In addition, in the embodiment shown in figure 3, the duodenal tube 12 is provided with a ring 19 of a radio opaque material, in particular a metal. The ring 19 is arranged in an area 21 of the duodenal tube 12 which is distant from the proximal end 20 of the duodenal tube 12. This helps positioning the device under x-ray control.

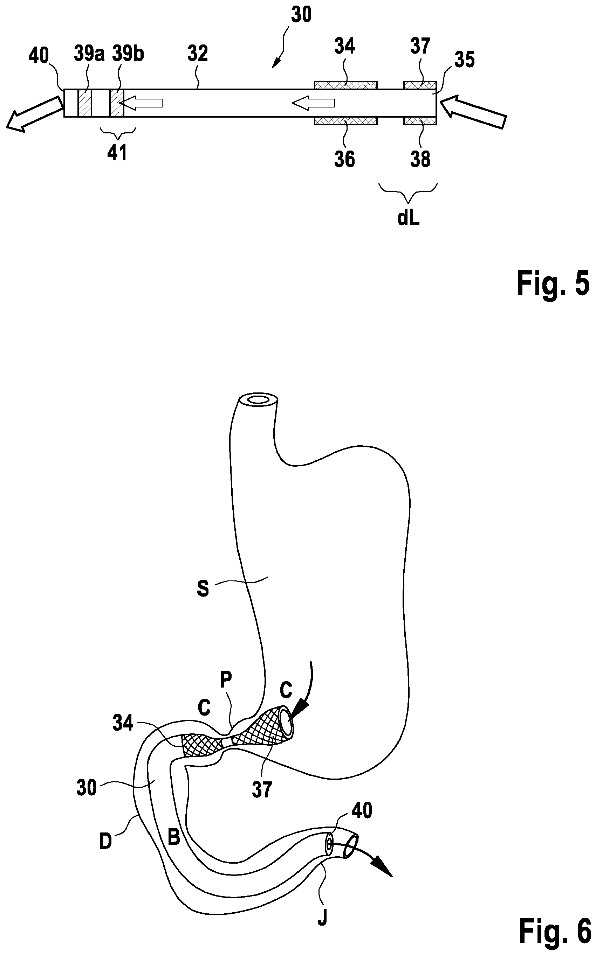

Figure 5 shows an embodiment of the invention which is suitable for treatment of diabetic patients. The device 30 according to figure 5 also comprises a duodenal tube 32. The duodenal tube 32 is provided with a first anchor 34 and a second anchor 37. The second anchor 37 is arranged neighbouring the proximal end 35 of the duodenal tube 32 whereas the first anchor 34 is arranged at a distance d of the proximal end 35. The first anchor 34 and the second anchor 37 both are formed of expandable structures 36, 38 of braided nitinol wire, respectively.

The duodenal tube 32 is provided with two rings on its outer surface: a first ring 39a is arranged at the distal end 40 whereas a second ring 39b is arranged in an area 41 which is distant from the distal end 40. Similar as in the embodiment of figure 3, the duodenal tube 32 of figure 5 is made of polyurethane. The anchors 34, 37 and the rings 39a, 39b are mounted on the duodenal tube by gluing. It is, however, also conceivable to integrate in particular the rings 39a, 39b between multiple layers of the duodenal tube.

In the embodiment shown in figure 5, the second ring 39 is typically arranged at the distance of 10cm from the distal end 40.

Figure 6 shows the device 30 of figure 5 deployed within a patient. The first and second anchors 34, 37 are arranged on both sides of the pylorus and prevent distal or proximal migration of the device while still avoiding a direct contact with the tissue of the involved stomach S or the duodenum D. The anchor 37 is substantially smaller that the anchor 17 of the embodiment of Figures 3 and 4, making the device 30 of Figs. 5 and 6 less suitable for treatment of obesity, but suitable for diabetes treatment.

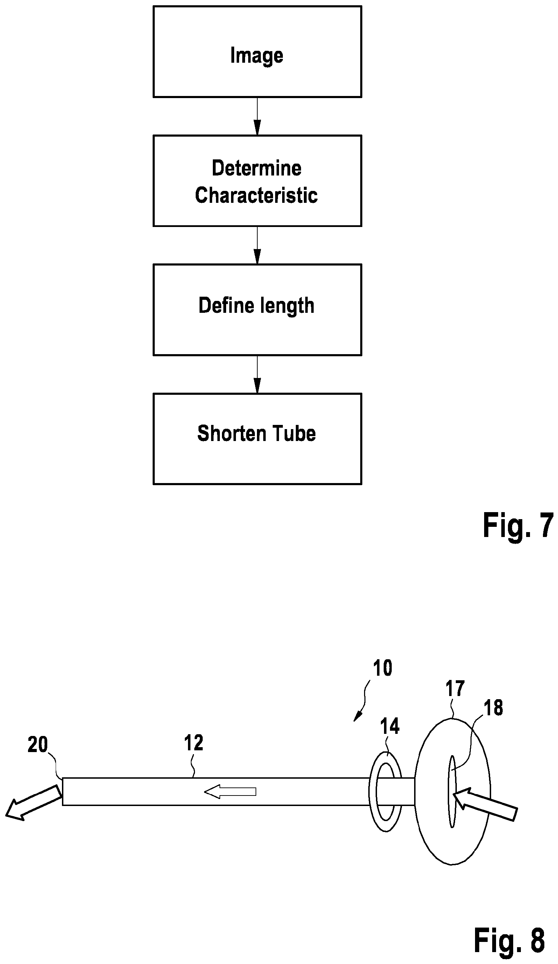

Figure 7 schematically shows various steps for selecting a device which is appropriate for an individual patient. In a first step "image" the patient is examined with examination methods known to the skilled person, in particular by diagnostic imaging. Based on this examination or imaging step, certain characteristics of the patient are then determined in step "Determine characteristic". Based on a CT scan, the thickness of the abdominal fat panicle, the fat mass surrounding the abdominal cavity and the visceral fat mass is determined. In addition, the body surface area, the body mass index and the abdominal perimeter are clinically defined. Additionally, it is also possible to define by way of CT scan the fat inside and/or outside the abdominal cavity. Based on these six or seven objective criteria, the length of the duodenal tube is defined in step "Define length". The length is typically between 450 and 600mm. As a general rule and by way of example, for more severe conditions of obesity, longer tubes will be chosen.

The first three criteria easily can be determined manually on the basis of an image shown on a display screen of a CT scanner based on a cross-section along the third lumbar vertebra. Of course these criteria also can be assessed fully or partly automatically by using artificial intelligent software.

In a final step "Shorten Tube", the tube is shortened to the defined length. Shortening made be made by cutting. For that purpose, markings may be provided on the outer surface of the duodenal tube (not specifically shown in figure 3). A ruler can be associated to the device.

Alternatively to the step of shortening the tube, a tube with an appropriate length may be chosen from a set of standard dimensions or may be individually manufactured.

Additionally or alternatively to the step of defining the length, also the inflation volume of the balloon like second anchor 17 may be determined based on the characteristic(s). By inflating the balloon to different volumes, an additional adaptation to an individual patient is possible. The amount to which the stomach should be filled and the size of the second anchor thereby may be defined.

Fig. 8 shows another embodiment of a device 10 similar to the one shown in Fig. 1. In the embodiment of Fig. 8, the first and the second anchor are formed by inflatable balloons 14, 17.

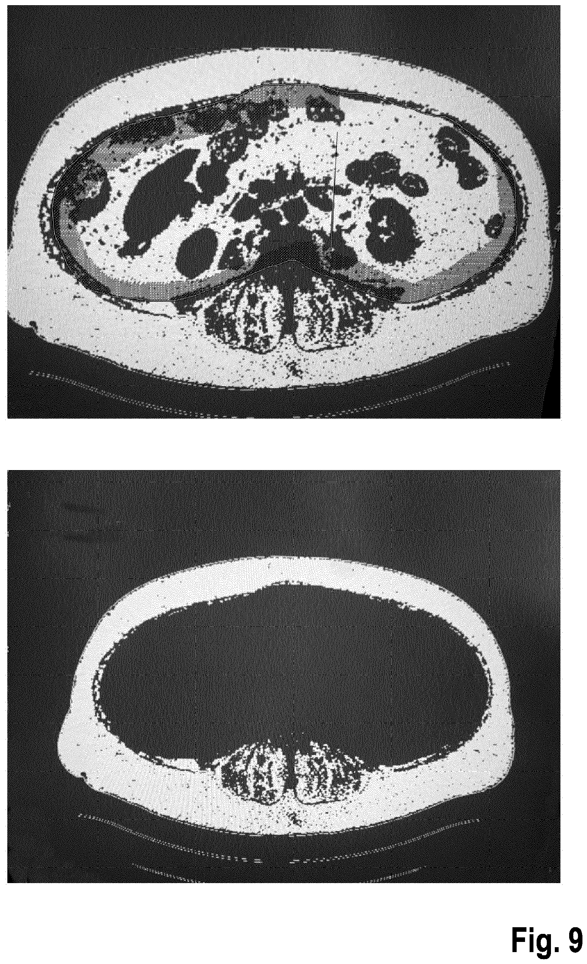

Fig. 9 shows two CT images. The abdominal fat is evaluated using the CT images and variations of the grey scale.

## Claims

1. A device (10;30) for treatment of obesity or diabetes of a patient, comprising a duodenal tube (12;32), adapted to be placed in the duodenum (D) and optionally jejunum (J) or ileum of a patient,
wherein a first anchor (14;34) is arranged at a predefined distance (d) from a proximal (15;35) end of the duodenal tube (12;32), adapted for anchoring the tube (12;32) distally to the pylorus (P), preferably without substantial mucosal involvement.

2. A device according to claim 1, wherein the first anchor (34) comprises an expandable structure (36), in particular a self-expandable structure and/or a balloon expandable structure.

3. A device according to claim 2, wherein the expandable structure (36) is made of a metal, preferably a shape memory metal, in particular Nitinol.

4. A device according to claim 3, wherein the self-expandable structure (36) is braided.

5. A device according to one of the claims 2 to 4, wherein the expandable (36) structure is covered by at least one layer of material, in particular a polymeric layer including or made of Dacron or polyurethane or a biologic layer, such as pericardial tissue.

6. A device according to one of the claims 1 to 5, where the first anchor and in particular the first expandable structure is retrievable and/or repositionable.

7. A device according to one of the claims 1 to 6, wherein the first anchor comprises patches attachable to the mucosa and linked to the duodenal tube via connectors, the patch preferably comprising a biocompatible adhesive and preferably being adapted to promote cell biocolonization.

8. A device according to one of the claim 1 to 7, wherein the duodenal tube (12;32) is provided with a second anchor (17;37) at its proximal end (15; 35), adapted to be positioned proximally of the pylorus (P) for anchoring the device in the stomach without substantial mucosal involvement.

9. A device according to claim 8 wherein the second anchor (17) is formed as an inflatable balloon (18), adapted to be positioned within the patient's stomach such as to reduce the gastric functional volume and shaped such as to avoid a tight contact with the inner wall of the stomach.

10. A device according to claim 9, wherein the balloon (18) has an annular shape surrounding the duodenal tube.

11. A device according to claim 10, wherein the balloon (18) has a crown shape.

12. A device according to one of claims 9 to 11, wherein the balloon (18) has a toroidal shape.

13. A device according to one of the claims 9 or 10, wherein the balloon (18) has, in the inflated condition in an area neighbouring the connection to the duodenal tube (12), a conical outer shape or a concave outer shape in a cross-section through a plane running through an axis of the device.

14. A device according to claim 9 or 10, wherein the balloon has a tulip shape in the area neighbouring the connection to the duodenal tube.

15. A device according to one of the claims 9 to 14, wherein the balloon (18) is adapted to be inflated to a volume of 200 ml to 800 ml, preferably 300 to 450 ml.

16. A device according to claim 8 wherein the second anchor (37) comprises a second expandable structure (38), in particular a self-expandable structure or a balloon expandable structure.

17. A device according to claim 8, 9, 10 or 16, wherein the second anchor has an at least partly hour-glass shape.

18. A device according to claim 16 or 17, wherein the second expandable structure (38) is made of a metal, preferably a shape memory metal, in particular Nitinol.

19. A device according to claim 16 to 18, wherein the second expandable structure (38) is braided.

20. A device according to one of the claims 16 to 19, wherein the second expandable structure (38) is covered by at least one layer of material, in particular a polymeric layer including or made of Dacron or a biologic layer, such as pericardial tissue.

21. A device according to one of the claim 1 to 20, wherein the device is provided with at least one additional element (19; 39a, 39b) on the duodenal tube, in particular a metallic element and/or a radiopaque element.

22. A device according to claim 21, wherein the at least one (19; 39a, 39b) element is formed as a ring mounted on at least one of a distal end (20;40) of the duodenal tube (12;32) and an area (21;41) distant from the distal end of the duodenal tube, preferably distant by 8 to 12 cm.

23. A device according to one of the claims 1 to 22, wherein the duodenal tube (12;32) is adapted to be shortened for adaptation to at least one characteristic of the patient.

24. A device according to one of the claim 1 to 23, wherein at least one of the duodenal tube, the first anchor and the second anchor is activatable by contact with contents of the intestine and/or stomach.

25. A device according to one of the claim 1 to 24, wherein the first anchor has an axial length of 1 cm to 3 cm.

26. A device according to one of the claim 8 to 25, wherein the second anchor (17;37) has an axial length of 2 cm to 10 cm.

27. A device according to one of the claims 1 to 26, wherein the duodenal tube (12;32) is made of a material which reduces a contact of nutrients migrating within the tube with the duodenal wall, in particular made of polyurethane.

28. A device according to one of the claims 1 to 27, wherein the duodenal tube (12;32) has a length in the range of 300 mm to 800 mm, in particular about 600 mm and/or a diameter of 25 to 35 mm, in particular about 28 mm.

29. A method for selecting a device for treatment of obesity or diabetes of a patient, in particular a device (10;30) according to one of the claim 1 to 28, comprising the steps of
- determining at least one characteristic of the patient selected from the group of thickness of the abdominal fat panicle, fat mass surrounding the abdominal cavity, visceral fat mass, the fat inside and/or outside the abdominal cavity, body surface area, Body Mass Index and abdominal perimeter, elasticity of the stomach, index of elasticity of the stomach, index of absorption for nutrients, measured on standing patient, at the umbilicus
- defining a length of a duodenal tube (12;32) on the basis of the selected characteristic
- providing a device (10;30) with a duodenal tube (12;32) having the defined length by one of (i) shortening the duodenal tube (12;32) to the defined length or (ii) selecting a device having a duodenal tube (12;32) with the defined length.

30. A method according to claim 29, comprising the further step of defining a filling volume of a gastric anchoring balloon based on the determined characteristic.

31. A method according to one of the claim 29 or 30, wherein the fat insideand/or outside the abdominal cavity is determined by Computerized Tomodensitometry.
